# EUROPÄISCHE PATENTSCHRIFT

(11) **EP 0 909 812 B1**
(45) Veröffentlichungstag und Bekanntmachung des Hinweises auf die Patenterteilung: **23.04.2003**
(21) Anmeldenummer: 98250275.9
(22) Anmeldetag: 30.07.1998
(51) Int. Cl.: C12N 1/20, B09C 1/10, C12S 9/00

(54) **Bakterienstämme P230 und P212a/2 und Verfahren zur mikrobiellen Dekontamination von Materialien, die mit Phenoxy- Herbiziden belastet sind**
Bacterial strains P230 and P212a/2 and methods for the microbial decontamination of materials polluted with phenoxy-herbicides
Souches bactériennes P230 et P212a/2 et procédures pour la décontamination microbienne de matériaux pollués par des herbicides phénoxy

(30) Priorität: 08.09.1997 DE 19740383
(43) Veröffentlichungstag der Anmeldung: 21.04.1999
(73) Patentinhaber: UFZ-UMWELTFORSCHUNGSZENTRUM LEIPZIG-HALLE GMBH, 04318 Leipzig (DE)
(72) Erfinder: Müller, Roland H. Dr., 04347 Leipzig (DE); Babel, Wolfgang, Prof.Dr., 04347 Leipzig (DE)
(74) Vertreter: Ziebig, Marlene, Dr. Dipl.-Chem.

(56) Entgegenhaltungen:
- WO-A-97/30758
- US-A- 4 535 061
- SMITH AE, ET AL.: "DEGRADATION OF MCPA, 2,4-D, AND OTHER PHENOXYALKANOIC ACID HERBICIDES USING AN ISOLATED SOIL BACTERIUM" JOURNAL OF AGRICULTURAL AND FOOD CHEMISTRY, Bd. 42, Nr. 2, 1994, Seiten 401-405, XP002118652
- MÜLLER RH, ET AL: "DEGRADATION VON PHENOXYALKANSÄURE-HERBIZIDEN DURCH ALKALIPHILE MOKROORGANISMEN - DEKONTAMINATION VON BELASTETEM BAUSCHUTT" HAMBURGER BERICHTE 10: NEUE TECHNIKEN DER BODENREINIGUNG,1996, Seiten 373-384, XP002118653 Bonn

## Beschreibung

Die Erfindung betrifft neue Bakterienstämme P230 (DSM11561) und P212a/2 (DSM11560) sowie ein Verfahren zur mikrobiellen Dekontamination von Materialien, die mit einzelnen oder Gemischen von Phenoxy-Herbiziden, wie z.B. 2,4-Dichlorphenoxypropionsäure (DCPP), 4-Chlor-2-methyl-phenoxypropionsäure (MCPP), 2,4-Dichlorphenoxyessigsäure (2,4-D), 4-Chlor-2-methylphenoxyessigsäure (MCPA), 2,4-Dichlorphenol (DCP) und/oder 4-Chlor-2-methylphenol und/oder deren Salzen belastet sind.

Sowohl Produktionsstandorte der chemischen Industrie, in denen jahrzehntelang produziert worden ist, wie auch deren Umfeld sind mit den Ausgangs-, Zwischen- und Endprodukten der Phenoxy-Herbizid-Produktion kontaminiert. Das betrifft sowohl die Böden, Oberflächenwässer und Grundwässer in solchen Arealen, aber auch die Produktionsanlagen und Gebäude selbst. Das gilt u.a. auch für ehemalige Standorte der Phenoxy-Herbizid-Produktion.

Die dadurch vorhandenen Verbindungen, wie z.B. DCPP und MCPP, 2,4-D, MCPA, DCP und MCP sind bekanntermaßen toxisch und haben z.T. cancerogene und teratogene Wirkung. Auch führt die Demontage von Chemieanlagen, in denen diese Verbindungen produziert wurden, und die Zerkleinerung des Mauerwerks über Schredderanlagen zu einem Bauschutt, der dekontaminiert werden muß, um diese schädlichen Verbindungen zu beseitigen und um damit gesundheitliche Schäden von exponierten Personen zu vermeiden und auch, um Flora und Fauna zu schützen.

Eine mikrobielle Dekontamination von Phenoxy-Herbiziden zeichnet sich, von einigen Ausnahmen abgesehen, dadurch aus, daß die Stämme eine hohe Substratspezifität betreffs der Phenoxy-Verbindungen aufweisen, die insbesondere durch die Alkansäurekette bestimmt ist. So sind dafür Stämme bekannt, die im Neutralen ihr Wirkungsoptimum haben und auch isolierte alkaliphile Mikroorganismen.

Die produktive Dekontamination von chlorierten und methylierten Phenolen und Phenoxyalkansäuren im neutralen pH-Bereich durch Einzelkulturen (Pieper, D.H. et al. 1988: Arch: Microbiol. 150, 95; Horvath, M. et al. 1990: Appl. Microbiol. Biotechnol. 33, 213; Kilpi, S. 1980: Microbiol. Ecol. 6, 261; Short, K.A. et al. 1990: Can. J. Microbiol. 36, 822; Tiedje, J.M. et al. 1969: J. Agr. Food Chem. 17, 1021) und Konsortien (Bloedorn, I. 1990: Dissertation, Universität Halle; Haugland, R.A. et al.1990: Appl. Env. Microbiel. 56, 1357: Lappin, H.M. 1985: Appl. Env. Microbiol. 49, 429; Oh, K.H. und Tuovinen, O.H. 1990: J. Ind. Microbiol. 6, 275) ist bekannt. Der Abbau von 2,4-Dichlorphenol sowie 4-Chlor-2-methylphenol durch ein gramnegatives Bakterium, Stamm S1, wurde ebenfalls beschrieben (Lechner et al., Biodegradation 6, 1995. 83).

In jüngster Zeit wurden Reinkulturen beschrieben, die zum Abbau von Phenoxyessigsäure-Derivaten in der Lage sind (Hoffmann et al. 1996: Acta Biotechnol 16, 121; Maltseva et al. 1996: Microbiology 142, 1115; Müller et al. 1996: Ist Internat. Symp. Extremophiles, Estoril, Portugal, p. 208).

Monokulturen sind jedoch im Vergleich zu Konsortien in ihrem metabolischen Spektrum eingeschränkt. Sie haben zwar den Vorteil der leichteren Handhabbarkeit, Kontaminationen aus der Herbizid-Produktion beinhalten aber oft ein Spektrum von Phenoxyalkansäure-Derivaten.

Bei Dekontaminationen mit Phenoxy-Herbizid-Mischungen ist deshalb die synergistische Wirkung unterschiedlicher Spezies erforderlich, um eine vollständige, produktive Mineralisierung aller Komponenten zu erzielen. Vollständiger Abbau eines komplexen Gemisches von substituierten Phenolen und Phenoxyalkansäuren in wäßrigen alkalischen Eluaten konnte durch Nutzung eines mikrobiellen Konsortiums mit nicht näher bestimmten individuellen metabolischen Aktivitäten erreicht werden (DE 44 24 756). Ein spezifischer Abbau einzelner Komponenten wurde durch den Einsatz spezieller Stämme erreicht (*Comamonas acidovorans P4a* für Phenoxyacetat-Derivate, DE 196 08 319; *Corynebacterium sp. K2-17* für die Spaltung von Phenoxybutyrat-Derivaten, DE 196 54 624). Bakterien, die zur Degradation von Phenoxypropionat-Herbiziden unter alkalischen Bedingungen in der Lage sind, sind bislang nicht beschrieben worden.

Der Erfindung lag deshalb zum einen die Aufgabe zugrunde, ein anwendungsfähiges Verfahren zur mikrobiellen Dekontamination von mit Phenoxypropionat-Herbiziden belasteten Materialien und Wässern zu entwickeln, zum anderen auch ein Verfahren bereitzustellen, das für den Abbau von mit Phenoxy-Herbizid-Gemischen belasteten Materialien geeignet ist, sowie Mikroorganismen zu finden, die für einen komplexen Abbau dieser Verbindungen geeignet sind.

Es wurden zwei neue Bakterienstämme gefunden, die sowohl einzeln als auch in Kombination zum Abbau von DCPP und/oder MCPP und deren Salzen geeignet sind und im schwach sauren bis alkalischen Milieu eingesetzt werden können. Darüber hinaus hat sich überraschend gezeigt, daß diese Stämme auch zum Abbau von weiteren Phenoxy-Herbiziden geeignet sind. Es werden neben den oben genannten Propionaten zusätzlich auch noch die Phenoxyacetat-Derivate (2,4-D, MCPA) sowie die entsprechenden substituierten Phenole (DCP, MCP) sowie deren Salze abgebaut. Dabei ist es unerheblich, ob nur eine dieser Verbindungen vorhanden ist oder alle genannten Verbindungen vorhanden sind. Es erfolgt eine simultane, vollständige Mineralisierung.

Es handelt sich um die Stämme P230 und P212a/2. Sie wurden aus Herbizid-belastetem Mauerwerk isoliert und beide am 21.05.1997 bei der Deutschen Sammlung von Mikroorganismen und Zellkulturen GmbH unter den Nummern DSM 11561 (P230) und DSM 11560 (P212a/2) hinterlegt.

Diese Stämme wachsen im neutralen bis alkalischen Bereich und sind überraschend in der Lage, sowohl DCPP und MCPP, als auch 2,4-D und MCPA und deren Salze in die entsprechenden Phenolderivate und C2- bzw. C3-Derivate zu spalten sowie die Phenolderivate zu mineralisieren, wobei der Abbau der einzelnen Komponenten auch simultan erfolgen kann.

Die Degradation der Phenoxy-Herbizide erfolgt unter aeroben Bedingungen im schwach sauren bis alkalischen Milieu bei pH-Werten im Bereich von 6 bis 12,5, bevorzugt im Bereich 8 bis 10, und bei Temperaturen zwischen 4 bis 38°C.

Die Kultivierung der Stämme P230 und P212a/2 erfolgt nach an sich bekannten Verfahren kontinuierlich, diskontinuierlich oder mittels anderer Fütterungsstrategien. Diese Kultivierung wird entweder getrennt oder im Gemisch durchgeführt. Die Dekontamination belasteter Materialien erfolgt durch die Bakterienstämme einzeln oder im Gemisch in Gegenwart an sich üblicher Wachstumskomponenten, wie z.B. Stickstoff und Phosphor. Stickstoff kann auch als N₂ über die Luft bereitgestellt werden.

Bevorzugt erfolgt die Dekontamination in wäßrigen Eluaten aus belasteten Materialien oder die belasteten Materialien liegen zur Dekontamination in befeuchtetem Zustand vor.

In einer bevorzugten Ausführungsvariante werden die Bakterienstämme ex situ bei pH-Werten zwischen 6 bis 11, vorzugsweise bei pH-Werten zwischen 8 bis 10, auf 2,4-D, MCPA, DCPP und/ oder MCPP und/oder deren Salzen sowie ggf. DCP und/oder MCP enthaltenden Materialien vermehrt.

Zur Stabilisierung des Wachstums und zur Erhöhung der Wachstumsrate wird bei der Vermehrung ggf. eine komplexe Kohlenstoff- und Energiequelle zugegeben, wie z.B. n-Alkansäuren, Dicarbonsäuren oder andere als heterotrophes Substrat geeignete Substanzen, z.B. Hexosen (Glukose, Fructose), Pentosen (Ribose, Arabinose, Xylose), Glycerol, Gluconsäure oder solche Verbindungen oder Gemische von Verbindungen enthaltende Abprodukte. Das Wachstumsmedium wird durch geeignete komplexe Substrate, z.B. Hefeextrakt supplementiert.

Die jeweiligen Verbindungen (2,4-D, MCPA, DCPP, MCPP, DCP und/oder MCPP) können somit direkt aus den belasteten Materialien vollständig abgebaut werden.

Bei kontinuierlicher Vermehrung des Stammes P230 und/oder 212a/2 werden gleichzeitig die belasteten Materialien, wie z.B. Böden und Wässer kontinuierlich dekontaminiert. Die kontinuierliche Vermehrung der Stämme auf einem entsprechenden Wachstumsmedium, welches gegebenenfalls durch eine komplexe Kohlenstoffquelle supplementiert ist, kann unter Nutzung eines geeigneten nicht chlororganischen heterotrophen Substrates als Kohlenstoff- und Energiequelle auch so erfolgen, daß dieses Substrat in Relation zur Herbizid-Komponente überproportional angeboten wird. Die Vermehrung der Stämme kann selbst ohne Zusatz der Herbizidkomponenten erfolgen. In diesem Falle kann die Biomasse durch Zusatz eines verwertbaren Herbizids oder einer verwertbaren Herbizidmischung in einer anschließenden Stufe konditioniert werden oder die Konditionierug erfolgt nach Zugabe der Biomasse zu den dekontaminierten herbizidhaltigen Medien oder Matrices.

In einer weiteren Ausführungsvariante erfolgt die Kultivierung der Stämme diskontinuierlich. Die Stämme P230 und/oder P212a/2 werden mit einer als heterotrophem Substrat geeigneten Substanz in Gegenwart eines an sich üblichen Nährmediums, das zur Erhöhung der Wachstumsrate dient, einzeln oder im Gemisch vermehrt. Das Nährmedium enthält z.B. Hefeextrakt als Supplement. Anschließend wird der jeweilige Stamm oder das Gemisch aus den beiden Stämmen durch Zugabe von 2,4-D, MCPA, DCPP und/oder MCPP und/oder deren Salzen und ggf. DCP und/oder MCP konditioniert.

Besonders bevorzugt wird diese Konditionierung der Stämme nach ihrer Vermehrung in situ auf einem 2,4-D, MCPA, DCPP und/oder MCPP, ggf. DCP und/oder MCP enthaltendem Medium oder Material durchgeführt, wodurch die Verbindungen abgebaut werden. Zur diskontinuierlichen Dekontamination wird Biomasse des Stammes P230 und/oder P212a/2 den belasteten Materialien oder Wässern zugesetzt, bevorzugt in einer Konzentration von 0,01 bis 1g pro Liter oder kg, wobei der pH-Wert zwischen 6 und 12,5 liegen und das kontaminierte Material eine totale Konzentration bis zu 2g/kg im wäßrigem Milieu aufweisen kann.

Das erfindungsgemäße Verfahren wird vorzugsweise im Festbettreaktor, z.B. mittels Mietentechnik oder als Flüssigphasenreaktor betrieben, wobei der Prozeß kontinuierlich, partiell kontinuierlich (z.b. fed- batch) oder diskontinuierlich betrieben werden kann. In Abhängigkeit vom gewählten Verfahrensregime können Maßnahmen zur Rückhaltung der Biomasse von Vorteil sein.

Unter den genannten Bedingungen sind die erfindungsgemäßen Stämme P230 und/oder P212a/2 einzeln und im Gemisch hervorragend geeignet, DCPP/ MCPP und/oder 2,4-D/MCPA unter intermediärer Bildung von DCP/MCP und deren weiterer vollständiger Mineralisierung zu dekontaminieren. Dadurch sind sie hervorragend zur wesentlichen Schadstoffbeseitigung von Materialien, die mit mehreren Phenoxy-Herbiziden belastet sind, einsetzbar.
Das erfindungsgemäße Verfahren wird vorteilhaft insbesondere zur Sanierung von Gebäudeabbruch, beispielsweise an Standorten der chemischen Industrie oder mit solchen Substanzen kontaminierten Arealen bzw. Lager- und Umschlagplätzen oder zur Behandlung von Wässern aus der Produktion solcher Verbindungen oder zur Behandlung von mit diesen Herbiziden verunreinigten Böden, Oberflächenwässern und Grundwässern angewendet.

Anschließend wird die Erfindung an Ausführungsbeispielen näher erläutert.

### Beispiel 1

Der Stamm P230 wurde auf einem Minimalmedium angezogen. Dieses Medium hat pro Gramm zu erzeugender Biomasse folgende Zusammensetzung (in mg/l): NH₄Cl, 700; KH₂PO₄ 158; MgSO₄*7 H₂O, 8; CaCl₂*2H₂O, 10; Fe SO₄*7 H₂O, 2,5; ZnSO₄*7 H₂O, 0,23; MnSO₄* 4 H₂O, 0,42; CuSO₄*5 H₂O, 0,39; Na₂MoO₄, 0,12. Die Kultivierung erfolgte bei pH 8,5, bei 30°C und einer Gelöstsauerstoffkonzentration von > 30% des Luftsättigungswertes. Als Kohlenstoff- und Energiequelle diente Na-Acetat. 2,4-D wurde simultan in einer Konzentration von 5% (w/w) bezüglich des Na-Acetats angeboten, desweiteren war das Medium mit 2,5% (w/w) Hefeextrakt supplementiert. Die Durchflußrate lag bei 0,07 h⁻¹. Die so erzeugte Biomasse wurde konzentriert und auf kontaminierten Bauschutt in einer Konzentration von 50 mg Bakterientrockenmasse pro kg Bauschutt aufgebracht. Der Bauschutt wurde mit einer Rate von 0,1 l/kg*h mit Wasser perkoliert. Das Material war mit einer Mischung von vor allem Phenoxyacetaten und Phenoxypropionaten kontaminiert, daneben fanden sich geringere Mengen von Phenoxybutyraten und chlorierten Phenolen. Die Konzentrationen der Hauptkontaminanten lagen im Bereich von 5 bis 10 mg/kg Bauschutt. Nach Zugabe der Biomasse erfolgte eine simultane Abnahme der Phenoxyacetat- und Phenoxypropionat-Derivate sowie der chlorierten Phenole im wäßrigen Eluat; nach ca. 14 Tagen lag die Konzentration dieser Verbindungen unterhalb der Bestimmungsgrenze für die HPLC. Die Belastung der festen Matrix wurde über den AOX-Wert erfaßt. Dieser Wert erreichte bereits nach 4 bis 6 Behandlungswochen Konzentrationen von ca. 20% des unbehandelten Ausgangsmaterials.

### Beispiel 2

Der Stamm P230 wurde diskontinuierlich auf dem in Beispiel 1 beschriebenen Minimalmedium mit Na-Acetat als alleiniger Kohlenstoff- und Energiequelle und Hefeextrakt als Supplement kultiviert. Die so erzeugte Biomasse wurde zur Dekontamination von mit Phenoxyalkansäure-Herbiziden und chlorierten/methylierten Phenolen belastetem Grundwasser eingesetzt. Die Konzentration der einzelnen Kontaminanten lag im Bereich von 1 bis 10 mg/l. Zugabe der Biomasse in einer Konzentration von 10 mg Bakterientrockenmasse pro l Grundwasser führte zu einer simultanen Abreicherung der Phenoxyacetat- und Phenoxypropionat-Verbindungen sowie der 2,4-dichlorierten und 4-chlor-2-methylsubstituierten Phenole innerhalb von 1 bis 2 Tagen auf über HPLC nicht mehr nachweisbare Konzentrationen. Durch Zugabe des in Patent DE 196 54 624 beschriebenen Stammes *Corynebacterium* sp. K2-17 mit Spezifität zur Spaltung der Phenoxybutyrat-Derivate konnte das Grundwasser von chlororganischen aromatischen Verbindungen im wesentlichen befreit werden.

## Patentansprüche

1. Bakterienstamm P230 (DSM 11561).

2. Bakterienstamm P212a/2 (DSM 11560).

3. Verfahren zur mikrobiellen Dekontamination von Materialien, die mit Verbindungen der Phenoxy-Herbizid-Produktion belastet sind,
**dadurch gekennzeichnet, daß** man
zur Dekontamination Bakterienstamm P230 (DSM 11561) und/oder Bakterienstamm P212a/2 (DSM 11560) im leicht sauren bis alkalischen Bereich unter aeroben Bedingungen bei Temperaturen zwischen 4 und 38 °C einsetzt.

4. Verfahren nach Anspruch 3,
**dadurch gekennzeichnet, daß** die herbiziden Phenoxy-Verbindungen 2,4-Dichlorphenoxypropionsäure (DCPP) und/oder 4-Chlor-2-methylphenoxypropionsäure (MCPP) sowie deren Salze sind.

5. Verfahren nach Anspruch 3 oder 4,
**dadurch gekennzeichnet, daß** die herbiziden Phenoxy-Verbindungen 2,4-Dichlorphenoxyessigsäure (2,4-D), 4-Chlor-2-methylphenoxyessigsäure (MCPA), 2,4-Dichlorphenol (DCP) und/oder 4-Chlor-2-methylphenol (MCP) sowie deren Salze sind.

6. Verfahren nach einem der Ansprüche 3 bis 5,
**dadurch gekennzeichnet, daß**
die Dekontamination in wäßrigen Eluaten aus den belasteten Materialien oder von solchen Materialien in befeuchtetem Zustand erfolgt.

7. Verfahren nach einem der Ansprüche 3 bis 6,
**dadurch gekennzeichnet, daß**
der Bakterienstamm oder die Bakterienstämme getrennt oder im Gemisch kontinuierlich, diskontinuierlich oder mittels anderer Fütterungsstrategien nach an sich üblichen Methoden kultiviert werden und anschließend zur Dekontamination in Gegenwart an sich üblicher Wachstumskomponenten eingesetzt werden, wobei die Dekontamination von dem mit DCPP, MCPP, mit 2,4-D, MCPA, mit DCP und/oder MCP kontaminierten Material kontinuierlich, partiell kontinuierlich oder diskontinuierlich erfolgt.

8. Verfahren nach Anspruch 7,
**dadurch gekennzeichnet, daß**
die Bakterienstämme ex situ bei pH-Werten von 6-11, vorzugsweise von 8 bis 10, auf 2,4-D, MCPA, DCPP und/oder MCPP, und auf gegebenenfalls DCP und/oder MCP enthaltenden Materialien, vermehrt werden und die Vermehrung gegebenenfalls durch Zugabe einer komplexen Kohlenstoff- und Energiequelle oder einer anderen als heterotrophem Substrat geeigneten Substanz stabilisiert wird.

9. Verfahren nach einem der Ansprüche 3 bis 8,
**dadurch gekennzeichnet, daß**
die Vermehrung kontinuierlich erfolgt, wobei als Kohlenstoff- und Energiequelle ein geeignetes nicht chlororganisches Substrat, das die durch die Stämme verwertbaren Herbizide als Einzelkomponenten oder als Mischungen oder Kombinationen von beiden Substratkomponenten in variablem Verhältnis zueinander enthält, eingesetzt wird.

10. Verfahren nach einem der Ansprüche 3 bis 8,
**dadurch gekennzeichnet, daß**
die Stämme P230 und/oder P212a/2 diskontinuierlich vermehrt werden und in die Erzeugung der Biomasse ein Wachstumsschritt unter Nutzung eines an sich üblichen Nährmediums mit einer komplexen Kohlenstoff- und Energiequelle oder einer anderen als heterotrophem Substrat geeigneten Substanz mit einer anschließenden Konditionierung der Stämme unter Angebot von 2,4-D, MCPA, DCPP, MCPP, DCP und/oder MCP oder deren Salzen eingeschlossen ist.

11. Verfahren nach Anspruch 10,
**dadurch gekennzeichnet, daß**
die Konditionierung nach der Vermehrung in situ erfolgt, wodurch die Verbindungen in Gegenwart an sich üblicher Wachstumskomponenten abgebaut werden.

12. Verfahren nach Anspruch 9, 10 oder 11,
**dadurch gekennzeichnet, daß**
die Dekontamination diskontinuierlich erfolgt, indem Biomasse von den Stämmen P230 und/oder P212a/2 belasteten Materialien oder Wässern in einer Konzentration von 0,01 bis 1 g pro Liter oder kg zugesetzt wird.

13. Verfahren nach einem der Ansprüche 3 bis 12,
**dadurch gekennzeichnet, daß**
die Dekontamination in einem Festbettreaktor oder einem Flüssigphasenreaktor erfolgt.

## Claims

1. Bacterial strain P230 (DSM 11561).

2. Bacterial strain P212a/2 (DSM 11560).

3. A process for the microbial decontamination of materials polluted with compounds from the phenoxy herbicide production,
**characterized in that**
the bacterial strain P230 (DSM 11561) and/or the bacterial strain P212a/2 (DSM 11560) in a range of from slightly acidic to alkaline under aerobic conditions at temperatures between 4 and 38°C is employed in said decontamination.

4. The process according to claim 3,
**characterized in that**
said herbicidal phenoxy compounds are 2,4-dichlorophenoxypropionic acid (DCPP) and/or 4-chloro-2-methylphenoxypropionic acid (MCPP) and salts thereof.

5. The process according to claim 3 or 4,
**characterized in that**
the herbicidal phenoxy compounds are 2,4-dichlorophenoxyacetic acid (2,4-D), 4-chloro-2-methylphenoxyacetic acid (MCPA), 2,4-dichlorophenol (DCP), and/or 4-chloro-2-methylphenol (MCP), and salts thereof.

6. The process according to any of claims 3 to 5,
**characterized in that**
decontamination is performed in aqueous eluates from polluted materials or on such materials in wetted condition.

7. The process according to any of claims 3 to 6,
**characterized in that**
the bacterial strain or the bacterial strains are cultured separately or in admixture in a continuous or discontinuous fashion, or by means of other feeding strategies according to *per se* usual methods, and subsequently employed for decontamination in the presence of *per se* usual growth components, the decontamination of the material contaminated with DCPP, MCPP, with 2,4-D, MCPA, with DCP and/or MCP being effected in a continuous, partially continuous or discontinuous fashion.

8. The process according to claim 7,
**characterized in that**
the bacterial strains are grown *ex situ* at pH values of 6-11, preferably 8-10, on materials containing 2,4-D, MCPA, DCPP and/or MCPP and possibly DCP and/or MCP, said growth optionally being stabilized by adding a complex source of carbon and energy or another substance suitable as heterotrophic substrate.

9. The process according to any of claims 3 to 8,
**characterized in that**
growth is effected continuously, a suitable non-chloroorganic substrate being used as source of carbon and energy, which substrate contains the herbicides utilizable by the strains as single components or as mixtures or combinations of both substrate components at varying ratios with respect to each other.

10. The process according to any of claims 3 to 8,
**characterized in that**
the strains P230 and/or P212a/2 are grown discontinuously, the production of biomass comprising a growth step utilizing a *per se* usual nutrient medium including a complex source of carbon and energy or another substance suitable as heterotrophic substrate, with subsequent conditioning of the strain, supplying 2,4-D, MCPA, DCPP, MCPP, DCP, and/or MCP, or salts thereof.

11. The process according to claim 10,
**characterized in that**
said conditioning is performed *in situ* following growth, thereby degrading the compounds in the presence of per se usual growth components.

12. The process according to claims 9, 10 or 11,
**characterized in that**
decontamination is effected discontinuously by adding biomass of the strains P230 and/or P212a/2 to polluted materials or waters at a concentration of from 0.01 to 1 g per liter or kg.

13. The process according to any of claims 3 to 12,
**characterized in that**
decontamination is performed in a fixed-bed reactor or in a liquid-phase reactor.

## Revendications

1. Souche bactérienne P230 (DSM 11561).

2. Souche bactérienne P212a/2 (DSM 11560).

3. Procédé de décontamination microbienne des matériaux pollués par des composés issus de la production d'herbicides phénoxy,
**caractérisé en ce**
**qu'**on utilise pour la décontamination la souche bactérienne P230 (DSM 11561) et/ou la souche bactérienne P212a/2 (DSM 11560) dans une gamme de légèrement acide à alcaline, dans des conditions d'aérobie, à des températures comprises entre 4 et 38° C.

4. Procédé selon la revendication 3,
**caractérisé en ce que** les composés phénoxy herbicides sont l'acide 2,4-dichlorophénoxypropionique (DCPP), et/ou l'acide 4-chloro-2-méthylphénoxypropionique (MCPP) ainsi que leurs sels.

5. Procédé selon la revendication 3 ou 4,
**caractérisé en ce que** les composés phénoxy herbicides sont l'acide 2,4-dichlorophénoxyacétique (2,4-D), l'acide 4-chloro-2-méthylphénoxyacétique (MCPA), le 2,4-dichlorophénol (DCP) et/ou le 4-chloro-2-méthylphénol (MCP) ainsi que leurs sels.

6. Procédé selon une des revendications de 3 à 5,
**caractérisé en ce que**
la décontamination s'effectue dans des éluats aqueux des matériaux contaminés ou dans de tels matériaux à l'état humide.

7. Procédé selon une des revendications de 3 à 6,
**caractérisé en ce**
**qu'**on cultive la souche bactérienne ou les souches bactériennes de manière séparée ou en mélange, de manière continue, discontinue, ou par d'autres stratégies de nutrition selon des méthodes usuelles en soi, et qu'on les utilise ensuite pour la décontamination en présence de composants de croissance habituels en soi, la décontamination du matériau contaminé par du DCPP, du MCPP, du 2,4-D, du MCPA, du DCP et/ou du MCP s'effectuant de manière continue, partiellement continue ou discontinue.

8. Procédé selon la revendication 7,
**caractérisé en ce que**
les souches bactériennes prolifèrent *ex situ* à un pH de 6 à 11, de préférence de 8 à 10, sur des matériaux contenant du 2,4-D, du MCPA, du DCPP et/ou du MCPP, et éventuellement du DCP et/ou du MCP, et que la prolifération est éventuellement stabilisée par ajout d'une source de carbone ou d'énergie complexe ou d'une autre substance appropriée à titre de substrat hétérotrophe.

9. Procédé selon une des revendications 3 à 8,
**caractérisé en ce que**
la prolifération s'effectue de manière continue, en utilisant en tant que source de carbone et d'énergie un substrat non organochloré approprié à cet effet, qui contient des herbicides utilisables par les souches en tant que composants uniques ou en tant que mélanges ou combinaisons des deux composants de substrat dans un rapport variable de l'un à l'autre.

10. Procédé selon une des revendications 3 à 8,
**caractérisé en ce que**
les souches P230 et/ou P212a/2 prolifèrent par un processus discontinu et, que dans la production de la biomasse, est incluse une phase de croissance en utilisant un milieu nutritif habituel en soi, comportant une source de carbone et d'énergie complexe ou une autre substance appropriée en tant que substrat hétérotrophe, avec un conditionnement ultérieur des souches en présence de 2,4-D, de MCPA, de DCPP, de MCPP, de DCP et/ou de MCP ou de leurs sels.

11. Procédé selon la revendication 10,
**caractérisé en ce que**
le conditionnement s'effectue après la prolifération *in situ*, par lequel les composés sont dégradés en présence de composants de croissance habituels en soi.

12. Procédé selon les revendications 9, 10, ou 11,
**caractérisé en ce que**
la décontamination s'effectue de manière discontinue, en ajoutant de la biomasse des souches P230 et/ou P212a/2 aux matériaux ou aux eaux polluées à une concentration de 0,01 à 1 g par litre ou par kg.

13. Procédé selon une des revendications de 3 à 12,
**caractérisé en ce que**
la décontamination s'effectue dans un réacteur à lit fixe ou un réacteur à lit fluidisé.
